# EUROPEAN PATENT SPECIFICATION

(11) **EP 0 809 653 B1**
(45) Date of publication and mention of the grant of the patent: **26.09.2001**
(21) Application number: 96902390.2
(22) Date of filing: 15.02.1996
(51) Int. Cl.: C07K 14/205, C07K 7/06, G01N 33/569, A61K 39/106

(54) **HELICOBACTER PYLORI ANTIGEN**
HELICOBACTER PYLORI ANTIGEN
ANTIGENE D'HELICOBACTER PYLORI

(30) Priority: 15.02.1995 GB 9502899
(43) Date of publication of application: 03.12.1997
(73) Proprietor: Provalis UK Limited, Deeside, Flintshire CH5 2NT (GB)
(72) Inventor: SMITH, Christopher, John, Clwyd LL17 0TE (GB); CLANCY, Robert, Llewellyn University of Newcastle, Newcastle Hospital Newcastle NSW 2300 (AU); CRIPPS, Allan, Belconnen, ACT 2616 (AU)
(74) Representative: Chapman, Paul William
(86) International application number: GB9600351
(87) International publication number: WO9625430

(56) References cited:
- EP-A- 0 329 570
- WO-A-93/22682
- FR-A- 2 669 929
- INFECTION AND IMMUNITY, vol. 60, no. 5, May 1992, WASHINGTON US, pages 2125-2127, XP002003032 D.J. EVANS, JR. ET AL.: "UREASE-ASSOCIATED HEAT SHOCK PROTEIN OF HELICOBACTER PYLORI."
- JOURNAL OF BACTERIOLOGY, vol. 173, no. 3, February 1991, WASHINGTON, D.C., US, pages 937-946, XP000570477 M. KOSTRZYNSKA ET AL.: "IDENTIFICATION, CHARACTERIZATION, AND SPATIAL LOCALIZATION OF TWO FLAGELLIN SPECIES IN HELICOBACTER PYLORI FLAGELLA."
- FEMS MICROBIOLOGY LETTERS, vol. 71, no. 1+2, 1 September 1990, AMSTERDAM, NL, pages 225-230, XP002003033 C,J, LUKE ET AL.: "IDENTIFICATION OF FLAGELLAR AND ASSOCIATED POLYPEPTIDES OF HELICOBACTER (FORMERLY CAMPYLOBACTER) PYLORI."

## Description

The present invention relates to a novel antigen of *Helicobacter pylori,* or antigenic fragments thereof, the use of the antigen or fragments thereof in detecting *Helictobacter pylori* and kits comprising them, as well as vaccines comprising the antigen or fragments thereof and a method for isolation of the antigen.

Gut infections in mammals, and in particular humans, stimulate an immune response in mucous secretions, such as saliva, through activation of the common mucosal immune system. This response often initially parallels an antibody response in serum although is generally characterised by the presence of IgA antibodies. However, the immune response in secretion, including saliva, rapidly diminishes following elimination of the antigen (eg bacteria or virus) from the body. Accordingly, the presence of antibody in mucous secretions reflects current, ie contemporary, infection. In the case of a microbial infection, for example, antibodies in mucous secretions, hereinafter referred to as secretious antibodies, reflect the current status of colonisation of the microbe, such as in the gut, and thus is a useful monitor of contemporary infection. Serum antibody, on the other hand, persists for some time after the microbe is eliminated from the body. A positive serum antibody test, therefore, reflects both past and present exposure to antigen which is less helpful to the clinician. A positive secretious antibody test, on the other hand, indicates present or contemporary infection by the microbe.

The diagnosis of *H. pylori* infection can be made by microscopy, microbiological culture or urease detection in gastric mucosal biopsies, urea breath test or by the presence of specific antibodies in serum ELISAs. It might be predicted that *H. pylori* infection, being an infection of the gastric mucosa, would elicit an IgA antibody response in gastric secretion. However, it has been discovered that *H. pylori*-specific antibody in mucous secretions is of the IgG class and not IgA as might have been expected. Little IgA antibody, if any, is detected. Accordingly, AU-A-9067676 is directed to the detection of IgG in mucous secretion specific to *H. pylori* antigen and thereby provides a means of monitoring current, ie contemporary, infection by that microorganism in mammals. The corresponding academic publication is Witt *et al*, *Frontiers in Mucosal Immunology* 1 693-696 (1991).

The presence of IgG antibodies in the saliva of *Helicobacter pylori* positive patients has received some attention in the proceedings of the Annual Meetings of the American Gastroenterological Association. After the disclosure by Czinn *et al* of the presence of such antibodies in the 1989 proceedings (Gastroenterology 96(5) Part2 (May 1989)) Larsen *et al* concluded in the May 1991 proceedings that salivary IgG levels are a practical, non-invasive marker of therapeutic response during a course of antibiotic therapy (Gastroenterology, 100(5), Page A107/May1991)). In the April 1992 proceedings, Landes *et al* confirmed earlier observations and observed that measurement of salivary IgG to *Helicobacter pylori* is a simple, non-invasive test for detecting *H. pylori* positive patients, especially in widespread or paediatric populations where other tests are not practical. (Gastroenterology, 102(4), Page A 107 (April 1992)). WO-A-9322682 discloses a convenient and reliable *in vitro* test for *H. pylori.* This test utilises an antigen preparation in a reaction with IgG antibody in a mucous secretion from a mammal being tested.

There is therefore a need to identify, isolate and thus provide novel antigens from *H*. *pylori* which can be used in diagnostic tests. These antigens should be specific, reliably purifiable, and should be characterised by good specificity and the lack of false positive results when used in such tests. In addition they may also form the basis of a vaccine useful either for the treatment or prophylaxis of *H.pylori* infection.

Various proteins derived from *H. pylori* have been isolated and characterised and examples of such proteins include those disclosed in Evans et al, Infection and Immunity, 60(5), 2125-2127, (1992), EP-A-0329570 and Kostrzynska et al, J. Bacteriol., 173(3)., 937-946 (1991). The present inventin relates to a novel antigenic protein derived from *H. pyroli* which can be used in diagnostic tests or from the basis of a vaccine.

Thus, in a first aspect, the present invention provides a protein being an *H. pylori* antigen and having a molecular weight in the range 55-65 kDa, as determined under denaturing and reducing conditions.

Suitably, the antigenic protein has, at its amino terminal end, the following amino acid sequence: 
M V T L I N N E D D
Met-Val-Thr-Leu-Ile-Asn-Asn-Glu-Asp-Asp.
 or a sequence substantially homologous thereto.

At the amino acid level, a protein sequence may be regarded as substantially homologous to another protein sequence if a significant number of the constituent amino acids exhibit homology. At least 40%, 50%, 60%, 70%, 80%, 90%, 95% or even 99%, in increasing order of preference, of the amino acids may be homologous.

It has also been found that parts of the whole protein are themselves antigenic. Thus, in a second aspect, the present invention provides an antigenic fragment of the protein of the invention.

In one preferred embodiment of this aspect of the invention, the antigenic fragment is a peptide having the sequence:

M   V   T    L   I   N   N   E
Met-Val-Thr-Leu-Ile-Asn-Asn-Glu

The skilled man will appreciate that some variation in the sequence of this fragment will be possible, while still retaining its antigenic properties. Such variants also form part of the invention.

The antigenic protein, or fragments thereof, of the present invention can be provided alone, as a purified or isolated preparation, or as part of a mixture with other *H. pylori* antigenic proteins.

In a third aspect, therefore, the invention provides an antigen composition comprising a protein of the invention and or one or more antigenic fragments thereof. Such a composition can be used for the detection and/or diagnosis of *H. pylori.* In one embodiment the composition comprises one or more additional *H. pylori* antigens.

In a fourth aspect, the present invention provides a method of detecting and/or diagnosing *H. pylori* which comprises:
(a) bringing into contact an antigenic protein, or antigenic fragment thereof, or an antigen composition of the invention with a sample to be tested; and
(b) detecting the presence of antibodies to *H. pylori*.

In particular, the protein, antigenic fragment thereof or antigen composition of the invention can be used to detect IgG antibodies. Suitably, the sample to be tested will be a biological sample, e.g. a sample of blood or saliva. An example of a suitable method for detection of *H.pylori* using a sample of a mucous secretion is that described in WO-A-9322682.

In a fifth aspect, the invention provides the use of an antigenic protein, antigenic fragment thereof or antigenic composition of the present invention in detecting and /or diagnosing *H. pylori.* Preferably, the detecting and/or diagnosing is carried out *in vitro.*

In a sixth aspect, the present invention provides a method of isolating an antigenic protein of the invention, which process comprises the following steps:
(a) preparing cultures of *H. pylori ,* growing the cultures under appropriate conditions and harvesting them, folowed by washing to yield a washed cell pellet;
(b) resuspending the washed cells in an appropriate buffer, followed by disruption of the cells;
(c) centrifugation to remove cell debris and obtaining the supernatant containing soluble cell proteins;
(d) subjecting the solution obtained to ion-change chromatography with a gradient elution, and assaying fractions thus obtained for the presence of urease;
(e) pooling urease containing fractions and subjecting said pool to gel permeation chromatography;
(f) selecting the appropriate peak; and
(g) confirming the presence of the specific protein having a molecular weight in the range 55-65 kDa in the isolate and isolating the protein.

The antigenic protein, antigenic fragment thereof or antigen composition of the invention can be provided as part of a kit for use in *in vitro* detection and/or diagnosis of *H. pylori.* Thus, in a seventh aspect, the present invention provides a kit for use in the detection and/or diagnosis of *H. pylori* comprising an antigenic protein, antigenic fragment thereof or antigen composition of the invention.

In addition, the antigenic protein or antigenic fragment thereof of the invention can be used to induce an immune response against *H. pylori.* Thus, in a further aspect, the present invention provides a composition capable of eliciting an immune response in a subject which comprises the protein or one or more antigenic fragments thereof of the invention. Suitably, the composition will be a vaccine composition, optionally comprising one or other suitable adjuvants. Such a vaccine composition may be either a prophylactic or therapeutic vaccine composition.

The vaccine compositions of the invention can include one or more adjuvants. Examples of adjuvants well known in the art include inorganic gels such as aluminium hydroxide or water-in-oil emulsions such as incomplete Freund's adjuvant. Other useful adjuvants will be well known to the skilled man.

In yet further aspects, the present invention provides:
(a) the use of the protein or one or more antigenic fragments thereof of the invention in the preparation of an immunogenic composition, preferably a vaccine;
(b) the use of such an immunogenic composition in inducing an immune response in a subject; and
(c) a method for the treatment or prophylaxis of *H. pylori* infection in a subject, which comprises the step of administering to the subject an effective amount of the protein, at least one antigenic fragment or an antigen composition of the invention, preferably as a vaccine.

Preferred features of each aspect of the invention are as for each other aspect *mutatis mutandis*.

The invention will now be described with reference to the following example which should not be construed as limiting the invention in any way.

### EXAMPLE 1

(a) Cultures of *H. pylori* were grown under appropriate conditions and the cells harvested into phosphate buffered saline. This was followed by repeated centrifugation to remove cell debris and other contaminants, for example agar, and fresh PBS was added three times to yield a washed cell pellet;
(b) The washed cells were resuspended in 0.1 M TRIS-HCl buffer pH 7.2 to be used in the ion exchange chromatography step. The cell suspension was then subjected to sonication (6*µ*m for 30 seconds, 60 seconds off, repeated 25 times for a 10ml sample containing cells from 100 agar plates) of sufficient intensity and duration to ensure disruption of the cells;
(c) The suspension was then centrifuged to remove cell debris and the supernatant, containing soluble cell proteins, was obtained;
(d) The solution from step (c) was then subjected to fractionation by ion-exchange chromatography using a strong anion exchange resin such as MonoQ •⃝ or Q-Sepharose •⃝ (Pharmacia), using a gradient elution based on increasing the sodium chloride concentration of the elution buffer from 0 to 1.0 M in a predetermined manner. The fractions were then assayed for the presence of urease;
(e) The urease containing fractions were then pooled and were subjected to gel permeation chromatography using a resin with a cut-off range of 5 x 10³-5 x 10⁶ Da for globular protein;
(f) The appropriate peak was selected by:
   (i) carrying out a urease assay of all the fractions and identifying the protein peak containing the urease activity; and
   (ii) analysing all the fractions shown to be urease positive and the protein containing peaks immediately adjacent to the urease peak but of low molecular weight by spotting one microgram of protein from these fractions onto nitro-cellulose or an equivalent, drying and then determining their ability to react with *H. pylori* specific antibodies in serum or saliva samples from *H. pylori* positive individuals;
(g) The presence of the specific protein in the isolate was confirmed by subjecting it to polyacrylamide gel electrophoresis (PAGE) and Western Blotting, and analysing the blot using IgG from a pool prepared from human serum collected from *H. pylori* positive individuals. The PAGE was carried out under denaturing conditions, and the protein identified as having a molecular weight in the range 55-65 kDa was isolated.

### EXAMPLE 2

Rabbits were inoculated with the peptide MVTLINNE. Blood samples were assayed as described below to test for antibody response.

### Assay Method

To the blood samples was added 25 *µ*l of 1% Thiomersal solution per ml of blood (i.e. a conc of 25 *µ*l/ml) , the samples then being stored at -40°C prior to testing.

The assays were carried out using plates wherein each well is coated with 100 *µ*l of 5 *µ*g/ml *H. pylori* antigen (antigen preparation as described in WO-A-93/22682), followed by 300 *µ*l of 1% Byco A solution as a post coat.

The blood samples were diluted using CDL wash buffer to produce a range of dilutions.

| **CDL wash buffer:** | **quantities for 1 dm**^{**3**} |
|---|---|
| Tris | 12.11 g |
| 5M HCl | 15 ml |
| Distilled water | 400 ml |
| NaCl | 87.66 g |
| Thiomersal | 0.1 g |
| Tween 80 | 50 g |

i) add tris to distilled water;
ii) add 5M HCl until pH 7.80 at 20°C results;
(iii) add Thiomersal;
(iv) add NaCl;
(v) add Tween 80;
(vi) make up to final volume with distilled water.

A Swine anti-Rabbit horseradish peroxidase conjugate with ABTS (2,2-Azino-di-[3-ethyl-benzthiazolin-sulfonate]) diluted 1/25 with Peroxidase Citrate Buffer as substrate was used to detect bound antibody, green colour indicating a positive result.

| **Peroxidase citrate buffer:** | **quantities for 1 dm**^{**3**} |
|---|---|
| Distilled water | 700 ml |
| Citric acid | 23.0 g |
| NaOH | 100 ml |
| 30% H₂O₂ | 0.5 ml |
| Adjust pH to 4.0 with 1M NaOH. | |

The assay protocol was as follows:
1) 100 *µ*l of diluted sample added to each well;
2) incubate for 30 mins (with shaking);
3) wash x5 with CDL wash buffer and dry plate;
4) 100 *µ*l of Swine anti-Rabbit HRP added to each well;
5) incubate for 30 mins (with shaking);
6) wash x5 and dry plate;
7) 100 *µ*l of ABTS added to each well;
8) incubate for 30 minutes; and
9) read plate @ 414 nm.

### Results

The peak antibody response occurred around six months post-inoculation. Table 1 below shows the results over time for blood samples from two rabbits inoculated with the peptide. The data is for 1/100 diluted samples.

**TABLE 1**

| **ABSORBANCE** | | |
|---|---|---|
| **Months postinoculation** | **Rabbit 1** | **Rabbit 2** |
| 2 | 0.484 | 0.149 |
| 3 | 0.129 | 1.154 |
| 4 | 0.491 | 1.057 |
| 5 | 1.236 | 0.7255 |
| 6 | 1.656 | 0.899 |
| 7 | 0.89 | 0.847 |
| 8 | 0.4095 | 0.7275 |

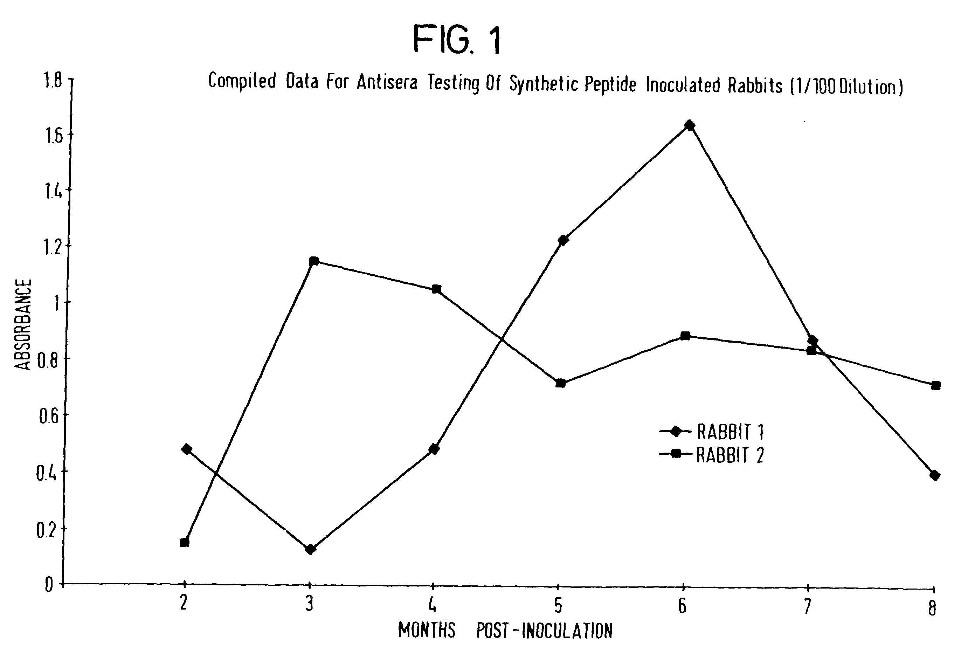

These results are shown in Figure 1.

Clearly, it can be seen that the peptide elicits an anti-*H*. *pylori* antibody response.

## Claims

1. A protein being an *H. pylori* antigen and having a molecular weight in the range 55-65 kDa, as determined under denaturing and reducing conditions, which has, at its amino terminal end, the sequence: Met-Val-Thr-Leu-Ile-Asn-Asn-Glu-Asp-Asp; or a sequence substantially homologous thereto.

2. An antigenic fragment of a protein as defined in claim 1.

3. An antigenic fragment as claimed in claim 2 having the sequence: 
 M   V   T   L   I   N   N   E
Met-Val-Thr-Leu-Ile-Asn-Asn-Glu
 or a sequence substantially homologous thereto.

4. An antigen composition comprising a protein as defined in claim 1 or at least one antigenic fragment as defined in claim 2 or claim 3.

5. An antigen composition as claimed in claim 4 which further comprises one or more other *H*. *pylori* antigens.

6. A protein as defined in claim 1, an antigenic fragment as defined in claim 2 or claim 3 or an antigen composition as defined in claim 4 or claim 5 for use in the detection and/or diagnosis of *H. pylori.*

7. A method of detecting and/or diagnosing *H. pylori* which comprises:
(a) bringing into contact a protein as defined in claim 1, at least one antigenic fragment as defined in claim 2 or claim 3 or an antigen composition as defined in claim 4 or claim 5 with a sample to be tested; and
(b) detecting the presence of antibodies to *H. pylori.*

8. A method as claimed in claim 7 wherein the sample is a sample of saliva.

9. The use of a protein as defined in claim 1, at least one antigenic fragment as defined in claim 2 or claim 3 or an antigen composition as defined in claim 4 or claim 5 in detecting and/or diagnosing *H. pylori.*

10. The method as claimed in claim 7 or claim 8 or the use as claimed in claim 9 wherein the detecting and/or diagnosing is carried out *in vitro*.

11. A method for isolating a protein as defined in claim 1 which comprises:
(a) preparing cultures of *H. pylori ,* growing the cultures under appropriate conditions and harvesting them, followed by washing to yield a washed cell pellet;
(b) resuspending the washed cells in an appropriate buffer, followed by disruption of the cells;
(c) centrifugation to remove cell debris and obtaining the supernatant containing soluble cell proteins;
(d) subjecting the solution obtained to ion-change chromatography with a gradient elution, and assaying fractions thus obtained for the presence of urease;
(e) pooling urease containing fractions and subjecting said pool to gel permeation chromatography;
(f) selecting the appropriate peak; and
(g) confirming the presence of the specific protein having a molecular weight in the range 55-65 kDa in the isolate and isolating the protein.

12. A kit for use in the detection and/or diagnosis of *H. pylori* comprising a protein as defined in claim 1, at least one antigenic fragment as defined in claim 2 or claim 3 or an antigen composition as defined in claim 4 or claim 5.

13. A composition capable of eliciting an immune response in a subject which comprises a protein as defined in claim 1, at least one antigenic fragments as defined in claim 2 or claim 3 or an antigen composition as defined in claim 4 or claim 5.

14. A composition as claimed in claim 13 which is a vaccine composition, optionally further comprising one or more adjuvants.

15. The use of a protein as defined in claim 1, at least one antigenic fragment as defined in claim 2 or claim 3 or an antigen composition as defined in claim 4 or claim 5 in the preparation of an immunogenic composition, preferably a vaccine.

## Patentansprüche

1. Protein, bei dem es sich um ein *H. pylori*-Antigen handelt und welches ein Molekulargewicht im Bereich von 55-65 kDa aufweist, wie unter Denaturier- und Reduzierbedingungen bestimmt wurde, und welches an seinem Aminoende die Sequenz: Met-Val-Thr-Leu-Ile-Asn-Asn-Glu-Asp-Asp oder eine dazu im wesentlichen homologe Sequenz aufweist.

2. Antigenes Fragment eines Proteins nach Anspruch 1.

3. Antigenes Fragment nach Anspruch 2, welches die Sequenz: 
M V T L I N N E
Met-Val-Thr-Leu-Ile-Asn-Asn-Glu
 oder eine dazu im wesentlichen homologe Sequenz aufweist.

4. Antigenzusammensetzung, umfassend ein in Anspruch 1 definiertes Protein oder zumindest ein antigenes in Anspruch 2 oder Anspruch 3 definiertes Fragment.

5. Antigenzusammensetzung nach Anspruch 4, welche weiters ein oder mehrere andere *H. pylori*-Antigene umfaßt.

6. Protein nach Anspruch 1, antigenes Fragment nach Anspruch 2 oder Anspruch 3 oder Antigenzusammensetzung nach Anspruch 4 oder Anspruch 5 zur Verwendung bei der Feststellung und/oder Diagnose von *H. pylori*.

7. Verfahren zum Feststellen und/oder Diagnostizieren von *H. pylori,* welches umfaßt:
(a) Inkontaktbringen eines in Anspruch 1 definierten Proteins, zumindest eines antigenen in Anspruch 2 oder Anspruch 3 definierten Fragments oder einer in Anspruch 4 oder Anspruch 5 definierten Antigenzusammensetzung, mit einer zu prüfenden Probe; und
(b) Feststellen des Vorliegens von Antikörpern auf *H*. *pylori.*

8. Verfahren nach Anspruch 7, wobei es sich bei der Probe um eine Speichelprobe handelt.

9. Verwendung eines in Anspruch 1 definierten Proteins, zumindest eines antigenen in Anspruch 2 oder Anspruch 3 definierten Fragments oder einer in Anspruch 4 oder Anspruch 5 definierten Antigenzusammensetzung beim Feststellen und/oder Diagnostizieren von *H. pylori.*

10. Verfahren nach Anspruch 7 oder Anspruch 8 oder Verwendung nach Anspruch 9, wobei das Feststellen und/oder Diagnostizieren *in vitro* erfolgt.

11. Verfahren zum Isolieren eines Proteins nach Anspruch 1, welches umfaßt:
(a) Anlegen von Kulturen von *H. pylori,* Züchten der Kulturen in geeigneten Bedingungen und Emten derselben, gefolgt von Waschen, um ein gewaschenes Zellpellet zu erhalten;
(b) Resuspendieren der gewaschenen Zellen in einem geeigneten Puffer, gefolgt vom Zerschlagen der Zellen;
(c) Zentrifugieren, um Zelltrümmer zu entfernen, Gewinnen des Überstandes, welcher lösliche Zellproteine enthält;
(d) Unterziehen der erhaltenen Lösung einer Ionenaustauschchromatographie mit einer Gradientenelution und Analysieren der auf diese Weise erhaltenen Fraktionen auf die Gegenwart von Urease;
(e) Vereinigen der ureasehaltigen Fraktionen und Unterziehen der vereinigten Fraktionen einer Gel-Permeationschromatographie;
(f) Auswählen der geeigneten Spitze; und
(g) Bestätigen der Gegenwart des spezifischen Proteins, welches ein Molekulargewicht im Bereich von 55-65 kDa im Isolat aufweist, und Isolieren des Proteins.

12. Set zur Verwendung bei der Feststellung und/oder Diagnose von *H. pylori,* umfassend ein in Anspruch Idefiniertes Protein, zumindest ein antigenes in Anspruch 2 oder Anspruch 3 definiertes Fragment oder eine in Anspruch 4 oder Anspruch 5 definierte Antigenzusammensetzung.

13. Zusammensetzung, welche in der Lage ist, eine Immunantwort in einem Subjekt hervorzurufen, und welche ein in Anspruch 1 definiertes Protein, zumindest ein antigenes in Anspruch 2 oder Anspruch 3 definiertes Fragment oder eine in Anspruch 4 oder Anspruch 5 definierte Antigenzusammensetzung umfaßt.

14. Zusammensetzung nach Anspruch 13, welche eine Impfstoffzusammensetzung ist, die wahlweise weiters einen oder mehrere Hilfsstoffe umfaßt.

15. Verwendung eines in Anspruch 1 definierten Proteins, zumindest eines antigenen in Anspruch 2 oder Anspruch 3 definierten Fragments oder einer in Anspruch 4 oder Anspruch 5 definierten Antigenzusammensetzung bei der Herstellung einer immunogenen Zusammensetzung, vorzugsweise eines Impfstoffes.

## Revendications

1. Protéine qui est un antigène de la souche *H. pylori* et a un poids moléculaire dans la plage de 55-65 kDa, comme déterminé dans des conditions de dénaturation et de réduction, qui a, à son extrémité de terminaison amino, la séquence : Met-Val-Thr-Leu-Ile-Asn-Asn-Glu-Asp-Asp; ou une séquence qui lui est sensiblement homologue,

2. Fragment antigénique d'une protéine selon la revendication 1.

3. Fragment antigénique selon la revendication 2 ayant pour séquence : 
M V T L I N N G
Met-Val-Thr-Leu-Ile-Asn-Asn-Glu;
 ou une séquence qui lui est sensiblement homologue.

4. Composition d'antigène comprenant une protéine selon la revendication 1 ou au moins un fragment antigénique selon la revendication 2 ou la revendication 3.

5. Composition d'antigène selon la revendication 4, qui comprend par ailleurs un ou plusieurs autres antigènes de la souche *H*. *pylori.*

6. Protéine selon la revendication 1, fragment antigénique selon la revendication 2 ou la revendication 3, ou composition d'antigène selon la revendication 4 ou la revendication 5, pour un usage dans la détection et/ou le diagnostic de la souche *H. pylori.*

7. Procédé de détection et/ou de diagnostic de la souche *H. pylori* qui comprend les étapes consistant :
(a) à amener en contact une protéine selon la revendication 1, au moins un fragment antigénique selon la revendication 2 ou la revendication 3 ou une composition d'antigène selon la revendication 4 ou la revendication 5, avec un échantillon à tester; et
(b) à détecter la présence d'anticorps à la souche *H. pylori.*

8. Procédé selon la revendication 7, dans lequel l'échantillon est un échantillon de salive.

9. Utilisation d'une protéine selon la revendication 1, d'au moins un fragment antigénique selon la revendication 2 ou la revendication 3 ou d'une composition d'antigène selon la revendication 4 ou la revendication 5 pour la détection et/ou le diagnostic de la souche *H. pylori.*

10. Procédé selon la revendication 7 ou la revendication 8 ou utilisation selon la revendication 9, dans lequel ou laquelle on effectue la détection et/ou le diagnostic *in vitro.*

11. Procédé pour isoler une protéine selon la revendication 1, comprenant les étapes consistant :
(a) à préparer des cultures de la souche *H. pylori,* à faire croître les cultures dans des conditions appropriées et à les récolter, le tout étant suivi d'un lavage pour obtenir une pastille cellulaire lavée;
(b) à remettre en suspension les cellules lavées dans un tampon approprié, le tout étant suivi d'une rupture des cellules;
(c) à centrifuger pour éliminer les débris cellulaires et obtenir le liquide surnageant contenant des protéines cellulaires solubles;
(d) à soumettre la solution obtenue pour procéder à une chromatographie par échange d'ions avec une élution à gradient, et à tester les fractions ainsi obtenues pour détecter la présence d'uréase;
(e) à combiner les fractions contenant de l'uréase et à . soumettre ladite combinaison à une chromatographie par perméation de gel;
(f) à choisir le pic approprié; et
(g) à confirmer la présence de la protéine spécifique ayant un poids moléculaire dans la plage de 55-65 kDa dans l'isolat et à isoler la protéine.

12. Trousse pour un usage dans la détection et/ou le diagnostic de la soucie *H. pylori* comprenant une protéine selon la revendication 1, au moins un fragment antigénique selon la revendication 2 ou la revendication 3 ou une composition d'antigène selon la revendication 4 ou la revendication 5.

13. Composition capable de susciter une réponse immunitaire chez un sujet, qui comprend une protéine selon la revendication 1, au moins un fragment antigénique selon la revendication 2 ou la revendication 3 ou une composition d'antigène selon la revendication 4 ou la revendication 5.

14. Composition selon la revendication 13, qui est une composition de vaccin, comprenant encore éventuellement un ou plusieurs adjuvants.

15. Utilisation d'une protéine selon la revendication 1, d'au moins un fragment antigénique selon la revendication 2 ou la revendication 3 ou d'une composition d'antigène selon la revendication 4 ou la revendication 5, dans la préparation d'une composition immunogène, de préférence un vaccin.
